# EUROPEAN PATENT SPECIFICATION

(11) **EP 2 295 096 B1**
(45) Date of publication and mention of the grant of the patent: **10.02.2016**
(21) Application number: 09170095.5
(22) Date of filing: 11.09.2009
(51) Int. Cl.: A61M 5/168, A61M 5/36, A61M 5/14

(54) **Micro-fluidic chambers for use in liquid medicament delivery systems**
Mikrofluidische Kammern zur Verwendung in Flüssigmedikament-Verabreichungssystemen
Chambres micro-fluidiques à utiliser dans des systèmes d'administration de médicaments liquides

(43) Date of publication of application: 16.03.2011
(60) Divisional application: 15198165.1
(73) Proprietor: F. Hoffmann-La Roche AG, 4070 Basel (CH); Roche Diagnostics GmbH, 68305 Mannheim (DE)
(72) Inventor: Geipel, Andreas, 4665, Oftringen (CH); Huwiler, Christoph, 6340, Baar (CH); Kühni, Florian, 3084, Wabern (CH)
(74) Representative: Rentsch Partner AG

(56) References cited:
- EP-A1- 0 718 016
- EP-A1- 1 818 664
- WO-A2-2004/085063
- US-A- 4 238 207
- US-A1- 2001 021 830

## Description

### Field of the Invention

The invention relates to micro-fluidic chambers for use in a liquid medicament delivery system, to pressure sensors, degassing devices, infusion pump devices, and liquid medicament delivery systems with such micro-fluidic chambers.

### State of the art

Devices for the automated release of liquid medicaments are normally used with patients who have a continuous and in the course of the day varying need of a medicine that can be administered by subcutaneous infusion. Specific applications are, for example, certain pain therapies and the treatment of diabetes. In such cases computer controlled infusion pump devices are used, which can be carried by the patient on the body, and which contain a certain amount of liquid medicament in a medicine reservoir. The medicine reservoir often comprises medicine sufficient for one or several days. The liquid medicament is supplied to the patient's body from the medicine reservoir through an infusion cannula or an injection needle.

Particularly in self-administration of medicaments, for example insulin, the patients using the medicament in question and administering it themselves by means of an infusion pump device emphasize convenience and discretion. As a consequence the acceptable dimensions of such infusion pump devices are limited, in order not be evident through clothing and to be carried as comfortable as possible. In an advantageous type of infusion pump device the liquid medicament is obtained by a downstream pump from a flexible container. Flexible containers have the advantage of a smaller volume surplus of the container in relation to its content, which reduces the manufacture costs and the achievable overall dimensions of an infusion pump device with such a flexible container.

A known problem of infusion pump devices are air bubbles in the fluidic system, particularly in the pump system, but also in other components, such as the container. If air bubbles remains in the fluidic system, they may be administered instead of the liquid medicament, which leads to potentially dangerous dosing errors. Furthermore the administration of air into a patient's body should be generally avoided for medical reasons.

Yet another problem resulting from air in the fluidic system is the reduced stiffness of the fluidic system, due to the high compressibility of gases in relation to liquids such as water. This impedes the detection of blockages or occlusions in the fluidic system by monitoring the fluidic pressure.

A further problem of fluidic systems, particularly in infusion pump devices, is the dead volume in the fluidic system. Said dead volume cannot be used, meaning that it cannot be emptied or drained completely. Thus the dead volume considerably increases the effective costs per dose and thus of the overall therapy costs, since a certain percentage of the liquid medicament inevitably remains in the fluid system and has to be disposed. This negative cost effect is particularly important for expensive medicaments. Furthermore the relative portion of a certain dead volume of a given overall reservoir size increases with decreasing absolute reservoir size. Minimizing the dead volume therefore becomes more and more important with decreasing reservoir size.

Micro-fluidic chambers are used in a variety of applications, for example as sensor chambers of pressure sensors for fluidic systems. Such pressure sensors typically comprise a chamber filled with liquid and fluidly connected to the fluidic system. The chamber is covered by a flexible, resilient membrane, such that a pressure difference between the fluidic pressure inside the sensor chamber and the outside (atmospheric) pressure will temporarily deform the membrane. The resulting deflection of the membrane can then be measured by suitable means, in order to determine the internal pressure of the fluidic system.

One approach to measure the deformation of the membrane is optical detection of a light beam reflected by the membrane. Such pressure sensors are for example disclosed in "A microfluidic experimental platform with internal pressure measurements", M. J. Kohl et al., Sensors and Actuators A 118 (2005), pp. 212-221.

Another approach is capacitive sensing, where the flexible, resilient membrane of the chamber acts as a capacitor electrode. When the membrane is deformed, the capacitance between the membrane capacitor electrode and a second capacitor electrode changes. Said capacitance is measured and used to determine the pressure difference acting on the membrane. US 2005/0243500 A1 discloses a pressure sensor where two capacitor electrodes are arranged on two opposite walls of a micro-fluidic chamber. When one of the walls is bulged inwards under an negative pressure difference, the change of capacitance is measured.

A further approach to measure the deformation of the membrane is the sue of strain gauges mounted to the membrane.

In the context of liquid medicament administration via an infusion pump device, pressure sensors as described above can be used for controlling the dosing, monitoring the correct operation of the system, and for fast detection of faults and hazards, such as occluded infusion lines or cannulae, empty containers, or malfunctioning pump systems. A pressure sensor is typically arranged in the fluid path downstream of a pump device and upstream of an infusion cannula. For such an application it is important that the micro-fluidic chamber of the pressure sensor is free of air bubbles, in order to avoid systematic or random measurement errors. Air bubbles in the in the micro-fluidic sensor chamber reduce the stiffness of the fluidic system, and thus delay the response of the sensor to pressure changes in the fluidic system. The resulting irreproducible measurement errors can reduce the dosing accuracy of an infusion pump device, and increase the response time to an occlusion event.

To avoid air bubbles in the micro-fluidic chamber when the fluidic system is filled the first time, the so called priming of the system, the chamber has to be filled in a controlled manner. However, this goal may be impeded by an uncontrolled orientation of the micro-fluidic chamber in space during this first filling procedure, since the gravitation field leads to buoyancy forces that act on the air bubbles. Depending on the orientation and the design of the micro-fluidic chamber, air bubbles may be caught in certain areas of the chamber.

Another field of use of micro-fluidic chambers are degassing devices for fluidic systems, particularly infusion pump devices, in which a liquid filled chamber is covered by a gas-permeable membrane. Subject to the condition that there is a positive difference between the partial pressure of the gas present in the fluidic system and the pressure on the opposite side of the permeable membrane, gas, as bubbles or solved in the liquid, can leave the fluidic system by permeating through the membrane. Also in such an application it is desirable that the properties of the micro-fluidic chamber and the performance of devices using such chambers are independent on the orientation of the micro-fluidic chamber with respect to the gravity field, since the orientation of the device during application is undefined and may constantly change.

For the present specification the meaning of the term "air" shall not only include air as such, but any gas or composition of gases that may be present in a fluidic system, particularly pure nitrogen or other protective gases.

EP 0718016 A1 discloses a liquid chromatography degassing device, comprising a sandwich-like stack formed by a number of identical supporting structures, between which gas-permeable membranes are arranged. The result is a filter having two separate chambers. In a first chamber, liquid solvent enters through an inlet, flows through a meandering channel, where it is degassed, and the degassed solvent leaves the chamber through an outlet. A second chamber is connected to a vacuum-pump. Gas in the solvent permeates through the membranes into the second chamber. The supporting structures comprise support ribs that have the same thickness than the supporting structure, and which form the meandering path of the fluid channel. Additional transverse ribs are arranged at right angles to the support ribs, and have a thickness that is less than that of the supporting structure. Both types of ribs support the membrane.

### Objects of the Invention

It is an object of this invention to provide a micro-fluidic chamber that overcomes the above-mentioned and other problems. Particularly such a micro-fluidic chamber should provide a smaller dead volume. It should be fillable without that air remains in the chamber. It should be producible at low cost in a large-scale manufacture.

Another object of the invention is to provide a micro-fluidic chamber that can be filled essentially independent on its orientation in space.

Yet another object of the invention is to provide an advantageous pressure sensor for use in a fluidic system, particularly in an infusion pump device for liquid medicaments.

A further object of the invention is to provide an advantageous degassing device for use in a fluidic system, particularly in an infusion pump device for liquid medicaments.

In addition it is an object of the present invention to provide an infusion pump device or parts of an infusion pump device, and liquid medicament delivery systems with such a micro-fluidic chamber, such a pressure sensor, and/or such a degassing device.

These and other objects are achieved by a micro-fluidic chamber, a pressure sensor, a degassing device, and an infusion pump device according to the independent claims. Advantageous embodiments are given in the dependent claims.

### Summary of the invention

A micro-fluidic chamber according to the invention for use in a liquid medicament delivery system comprises a bottom substrate and a top cover, the top cover being spaced from the bottom substrate so as to define a height of the chamber. One or more walls or fillings are positioned in the chamber, the walls or fillings defining a fluid channel there between, such that the fluid channel extends from an inlet of the chamber to an outlet of the chamber. Each of the walls or fillings has a height less than the height of the chamber so as to define a gap between a top surface of each wall or filling and the top cover. The dimensions of the walls or fillings and the chamber are chosen such that the fluid gap will be filled with liquid by capillary forces via the fluid channel when liquid is introduced into the fluid chamber. In other words, the fluid gap adjacent to a section of the fluid channel filled by a liquid introduced into the fluid chamber will be filled with said liquid by capillary forces. Preferably the top cover is a flexible, resilient membrane, and/or a gas-permeable membrane.

In a preferred embodiment of such a micro-fluidic chamber according to the invention, at least a part of the surface of the bottom structure, and/or the walls, and/or the top cover facing toward an inner volume of the chamber is hydrophilic. This increases the capillary forces in the gap, particularly for aqueous liquids.

Preferably the height of the gap lies between about 0.02 and about 0.2 mm, and more preferred between 0.05 and 0.15 mm. The fluid channel can have a curved or meander-like shape, or may be straight. Some embodiments of micro-fluidic chambers according to the invention have two or more fluid channels.

An additional conduit bypassing the micro-fluidic chamber according to the invention, by fluidly connecting an inlet conduit and an outlet conduit of the chamber provides the advantage of increasing the flow capacity of the micro-fluidic chamber. Preferably the width of an inlet of the bypass conduit is smaller than the width of the inlet conduit. This provides the advantage that air bubbles do not enter the bypass conduit.

In another advantageous variant of a micro-fluidic chamber according to the invention, one or more additional outlets conduits branch off from the fluid channel.

An advantageous pressure sensor according to the present invention for use in a liquid medicament delivery system comprises a micro-fluidic chamber according to the invention as discussed above. The pressure sensor can comprise an detection system that is arranged to measure the deformation of a top cover of the micro-fluidic chamber.

An advantageous degassing device according to the present invention for use in a liquid medicament delivery system also comprises a micro-fluidic chamber according to the invention.

An infusion pump device according to the present invention for use in a liquid medicament delivery system comprises a micro-fluidic chamber according to according to the invention. It may also comprise a pressure sensor and/or a degassing device according to the invention.

In a liquid medicament delivery system according to the present invention, a micro-fluidic chamber, and/or a pressure sensor according, and/or degassing device as discussed above are applied.

### Brief description of the drawings

In order to facilitate a fuller understanding of the present invention, reference is now made to the appended drawings. These references should not be construed as limiting the present invention, but are intended to be exemplary only.
- Figure 1: schematically shows a typical embodiment of a micro-fluidic chamber according to the invention, (a) in a top view, and (b) in a cross-section along plane A-A. Figure 1(c) shows a detail view of Figure 1(b).
- Figure 2: shows the distribution of liquid in an embodiment of a micro-fluidic chamber according to the invention, during the filling of the chamber in a real experiment, in two subsequent stages.
- Figure 3: depicts four other possible exemplary embodiments of micro-fluidic chambers according to the invention.
- Figure 4: discloses an advantageous embodiment of a micro-fluidic chamber according to the invention with a straight fluid channel, (a) in a top view, and (b) in a cross-section along plane A-A.
- Figure 5: discloses another advantageous embodiment of a micro-fluidic chamber according to the invention with a single wall arranged in the centre of the chamber.
- Figure 6: shows two variants of an optical detection scheme for the measurement of the displacement of the top membrane of a micro-fluidic chamber of a pressure sensor according to the invention.
- Figure 7: shows two variants of an capacitance detection scheme for the measurement of the displacement of the top membrane of a micro-fluidic chamber of a pressure sensor according to the invention.
- Figure 8: shows an embodiment of a degassing device according to the invention with a micro-fluidic chamber.
- Figure 9: shows (a) an embodiment of a micro-fluidic chamber according to the invention with additional outlets along the fluidic channel in the chamber, providing bubble-trap capabilities, and (b) an embodiment of a micro-fluidic chamber according to the invention with a bypassing additional conduit.

### Description of embodiments of the invention

### Micro-fluidic chamber

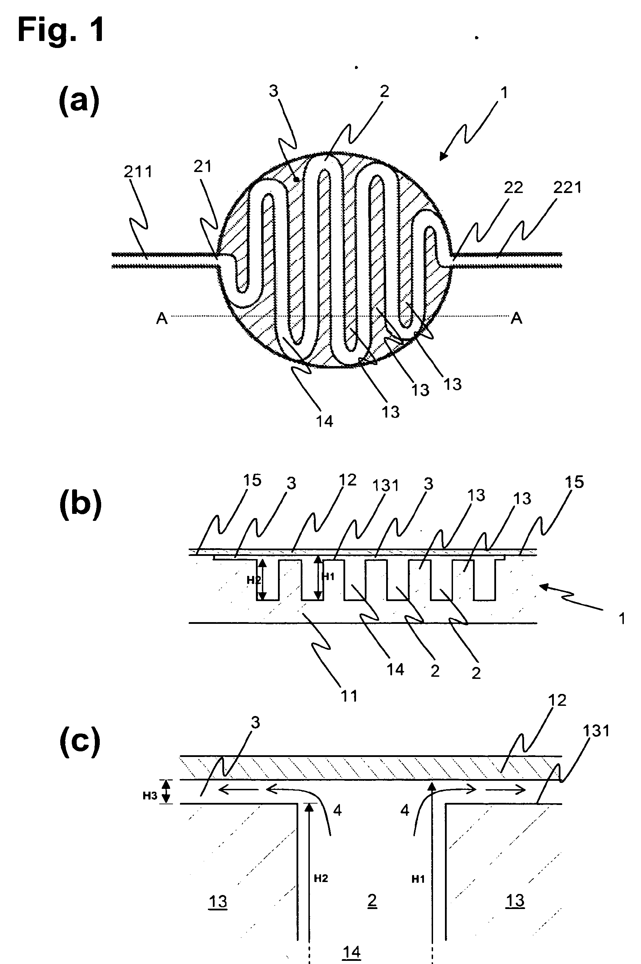

An advantageous embodiment of a micro-fluidic chamber according to the invention is shown in **Figure 1**. A circularly shaped fluid chamber 1 comprises a bottom substrate 11 and a top cover 12. The top cover 12 is spaced from the bottom substrate 11 by a certain height H1, thus defining an inner volume 14 of the chamber 1. Eight walls 13 are arranged in the fluid chamber 1, and define a meander-like fluid channel 12 that runs from an inlet 21 to an outlet 22 located on the opposite side of the chamber 1. Thus the inlet conduit 211 and the outlet conduit 221 are fluidly connected by the fluid channel 12.

The height H2 of the walls 13 is less that the overall height H1 of the chamber 1. As a result there is a fluid gap 3 between the top cover 12 and the upper surface 131 of the walls 13, with a height H3 = H1 - H2. The dimensions of the chamber and the walls, particularly the heights H1, H2, H3 are chosen such that there are non-negligible capillary forces acting on a fluid 4 present in the micro-fluidic chamber 1. Fluid 4 in the fluid channel 2 will be dragged by said capillary forces into the fluid gap 3.

The specific dimensions depend on one hand on the liquid used, and on the other hand on the properties the surfaces of the top cover 12 and the top 131 of the walls 13, since this will eventually define the interface tensions between liquid, surfaces, and gas/air in the chamber, which then will define the effective capillary forces for a certain geometric setting of a micro-fluidic chamber according to the invention. Since in most cases liquid medicaments are aqueous solutions, it is preferable that at least the most relevant surfaces, namely the surface of the top surface 131 of the wall 13 and the surface of the top cover 12 facing toward surface 131 are hydrophilic, with a contact angle < 90 °, in order to increase the overall capillary effect. For aqueous liquids a preferred range for the height H3 of the gap 3 lies between 20 and 200 µm, and preferably between 50 and 150 µm.

The dimensions of the chamber 1 and the fluid channel 2 are less critical. A typical diameter of a micro-fluidic chamber 1 may for example lie between about 2 to 10 mm. The fluid channels may have a width of for example 0.1 to 1 mm, while the height H2 of the walls 13 lies in a range between 0.25 to 5 mm, and preferred lies between 0.5 and 1 mm. The aspect ratio between the width of the fluid channel 2 and the height H2 can lie between 0.25 and 5, and is preferably about 1.

When a micro-fluidic chamber 1 according to the invention is filled through inlet 21 with a liquid 4, the liquid will flow essentially along the fluid channel 2. The capillary forces will drag liquid 4 in the fluid channel 2 into the adjacent sections of the gap 3, effectively supplanting air present in the gap. It is energetically much more favorable for air to form spherical bubbles with minimum surface toward the hydrophilic surroundings, and thus no air bubbles remaining in the gap 3.

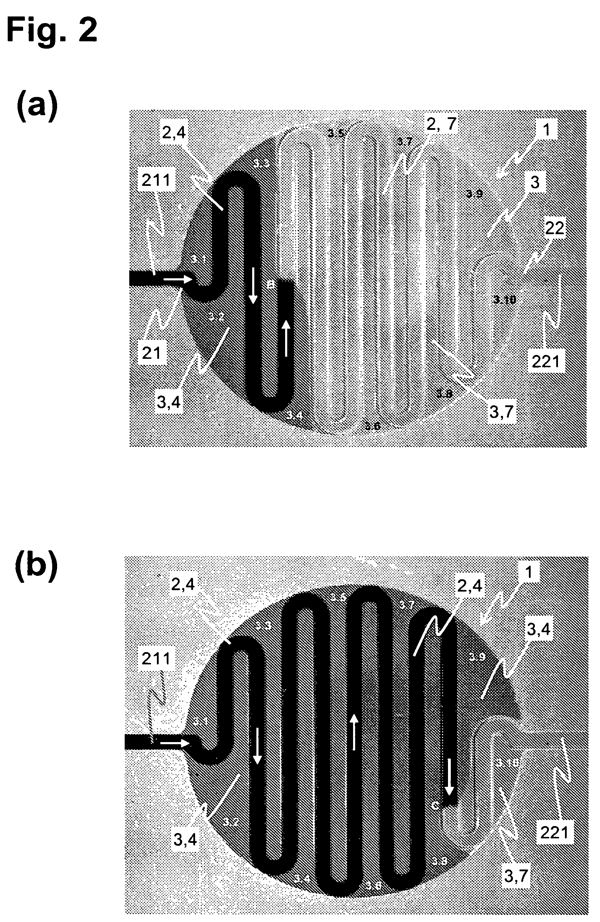

The first filling of a micro-fluidic chamber 1 according to the invention is demonstrated experimentally in **Figure 2**. In Figure 2(a) an aqueous liquid 4 has flown downstream through inlet conduit 211 and inlet 21 into the fluid channel 2, and is currently at a position B. Due to the capillary forces in the gap 3, the liquid 4 flows into the sections 3.1, 3.2 3.3, 3.4 of gap 3 adjacent to the fluid channel 2 already filled. In the gap the surrounding still empty sections of the fluid channel 2 downstream of position B limit the further flow of the liquid. Thus the gap 3 is filled section by section. Figure 2(b) shows a later stage, where the liquid 4 has proceeded in the fluid channel 2 to a position C. All sections of the gap 3 are filled, except section 3.10, which has not yet come into contact with the liquid 4. It is clearly visible in Figure 2 that no air remains in the part of the chamber that has already been filled by liquid. When finally the liquid will have reached the outlet 22 and the outlet conduit 221, the micro-fluidic chamber will be filled completely. No air bubbles remain in the chamber.

Air bubbles in the gap are energetically less preferable than air bubbles in the fluid channel 2. As a consequence also no air bubbles will form in the gap 3 at a later stage, and if they do they will migrate into the fluid channel 2. Air bubbles in the fluid channel 2, on the other hand, will not enter the gap 3 for energy reasons, but will be transported away by the liquid stream.

The shown capabilities of a micro-fluidic chamber 1 according to the invention are independent from its orientation in space. Since the capillary forces and interface tensions responsible for the smooth filling of the gap are much stronger than the gravitational force acting on the liquid, and the buoyancy force acting on the air bubbles in the liquid, the micro-fluidic chamber finally will be completely filled with liquid 4 independent on its orientation. Thus the filling behavior of such a micro-fluidic chamber are predictable and reproducible.

Since the operational internal volume of a micro-fluidic chamber is smaller than that of a hollow micro-fluidic chamber with similar dimensions as know from the art, the dead volume - the portion of the fluid volume in a fluid system that can never be drained and eventually will be lost - is considerably reduced.

A further advantage of a micro-fluidic chamber according to the invention is the fact that an air bubble that enters the chamber through the inlet will be guided through the fluid channel to the outlet. Since the effective cross-sectional area of the fluid channel is essentially constant over its length, the liquid flow is also constant over its length, and does not drop at certain positions. Thus bubbles cannot be caught in the fluid chamber.

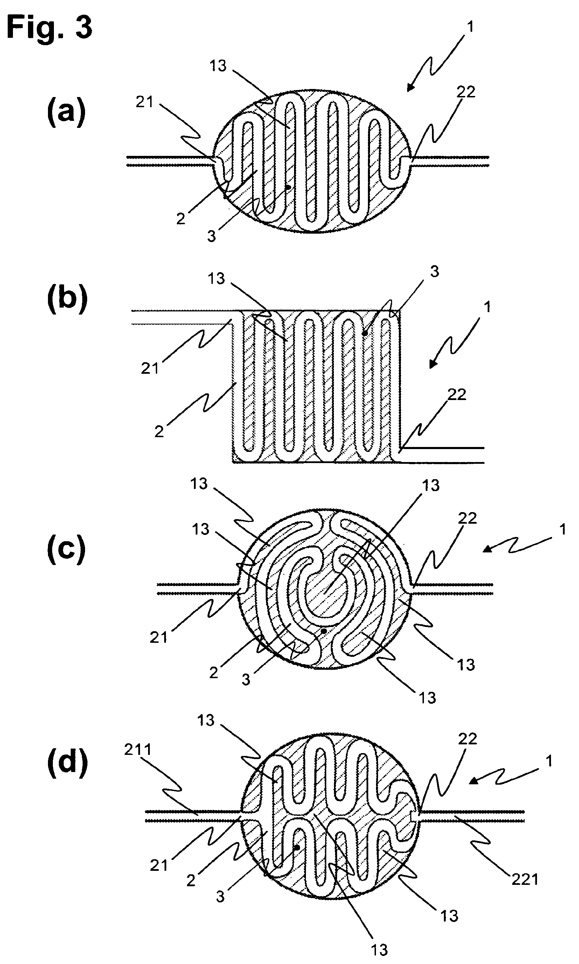

A micro-fluidic chamber according to the invention may have circular shape, as in the embodiments shown in Figures 1 and 2, or may have any other suitable shape. The same holds true for the specific design of the fluid channel in the chamber. Depending on the specific application of such a micro-fluidic chamber some embodiments may be preferable over others. **Figure 3** shows a number of possible variants of micro-fluidic chambers according to the invention. In Figure 3(a) the meanders of the fluid channel 2 are arranged in an elliptically shaped chamber 1, while in the embodiment of Figure 3(b) the chamber 1 has rectangular shape. In Figure 3(c) a circularly shaped chamber 1 with an alternative course of a meandering fluid channel 12 is shown.

Instead of having only one fluid channel 2, the walls 13 of a micro-fluidic chamber according to the present invention may define two or more fluid channels within the chamber, extending from a common inlet to a common outlet. Figure 3(d) shows such an embodiment of a micro-fluidic chamber 1. An inlet conduit 211 opens toward the chamber 1 through a common inlet 21. The fluid channel then splits up into two separate fluid channels 2, 2', which join again at a common outlet 22. In such an embodiment preferably constructive means such as flow barriers are provided that ensure that during the filling procedure the chamber 1 is completely filled before the liquid flow proceeds further through the outlet 22.

A curved or meandering design of the fluid channel is advantageous for fluid chambers with larger base areas, since the longest possible distance between the fluid channel and an outer edge of the gap is short. In addition the meandering fluid channel can be used as an efficient means to limit the maximum flow through a fluidic system.

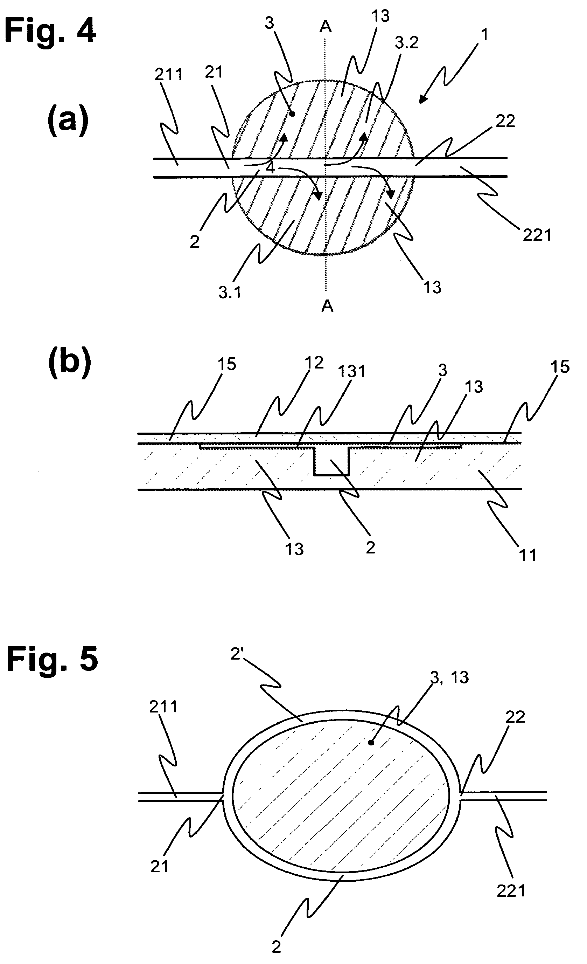

However, it is also possible to apply straight fluid channels for micro-fluidic chambers according to the invention. **Figure 4** shows such an embodiment of a micro-fluidic chamber 1 according to the invention. A straight fluid channel 2, arranged in a circularly shaped chamber 1, is in line with the inlet conduit 211 and the outlet conduit 221. Liquid 4 passing through the fluid channel 2 will be dragged into the two adjacent sections 3.1, 3.2 of the gap 3 on both sides of the fluid channel 2. Such an embodiment of a micro-fluidic chamber 1 has the advantage of a reduced overall inner volume of the chamber 1 in relation to the area of the gap 3, which is advantageous for certain applications. It also has a reduced flow resistance compared to a meander-shaped fluid channel.

Another possible variant of a micro-fluidic device 1 according to the invention is shown in **Figure 5**, where two fluid channels 2, 2' run from a common inlet 21 along the edge of the chamber 1 to a common outlet 22. A single wall or filling 13 is arranged in the centre of the chamber 1. The resulting central gap 3 is defined by the volume between the large circular top surface of the wall 13 and the top cover. As discussed for Figure 3(d) preferably constructive means such as a flow barriers are used to ensure a complete filling of the chamber.

In the embodiments of micro-fluidic chambers according to the invention discussed so far, the fluid chamber 1 comprises a bottom structure 11 and a top cover 12, which are sealed together in a sealing area 15 along an outer rim of the chamber 1. Suitable materials of the bottom substrate 11 and the top cover 12 are for example polymer materials. Suitable methods for connecting the two substrates 11, 12 are thermal bonding, laser bonding, gluing etc.

The walls 13 can be realized as an integral part of the bottom substrate 11. In such a case the fluid channel 2, and even the inlet and outlet conduits can, as an example, be produced by embossing the necessary void structures into a flat bottom structure 12. To obtain the necessary gap 3 one may arrange a thin spacer layer with height H3 between bottom layer structure and top layer 12 around the chamber, or may produce the gap together with the fluid channel and the walls in the embossing step. Another suitable technology for the manufacture of micro-fluidic chambers according to the invention is injection molding.

In a possible alternative approach the walls 13 are realized as separate filling structures, mounted onto a flat bottom layer 11. In that approach, a filling body may be attached to a bottom layer, and then may be arranged between said bottom layer and a adjacent top layer in a sandwich-like manner.

Since micro-fluidic chambers according to the invention may be manufactured in large numbers and on continuous production lines, the effective costs per piece are that low that they can be applied for disposable products, for example for parts of an infusion pump device that are disposed after use for hygienic reasons.

### Pressure sensor

An advantageous field of application for a micro-fluidic chamber according to the invention are pressure sensors for fluidic systems, particularly for pressure sensors for miniaturized pump systems such as infusion pump devices for liquid medicaments. A pressure sensor according to the present invention with a micro-fluidic chamber will comprise a rigid bottom structure 11 and a flexible, resilient membrane 120 as the top layer 12. When there is no pressure difference between the external pressure and the internal pressure of the fluidic system, the top layer membrane 120 remains flat. In the case of a positive pressure difference, the membrane 120 will bulge outwards. The resulting displacement of the outer surface of the flexible membrane 120 then is used to determine the current pressure difference. In the case of a negative pressure difference, where the flexible membrane 120 will displaced inwards toward the chamber 1, the walls 13 will support the membrane 120, thereby avoiding an occlusion of the micro-fluidic chamber 1 according to the invention, or even damage of the membrane 120. Another advantage of such a pressure sensor according to the invention with a micro-fluidic chamber is the considerably reduced dead volume of the pressure sensor compared to the state of the art.

In an alternative embodiment of a micro-fluidic chamber according to the invention for use in a pressure sensor, the roles of the bottom substrate and the top layer are reversed. In such a variant the top layer will be a rigid structure, while the bottom substrate is a flexible, resilient membrane. Thus the walls or fillings protrude from the flexible membrane. To measure the pressure in the fluid system, the deformation of the flexible bottom structure is measured.

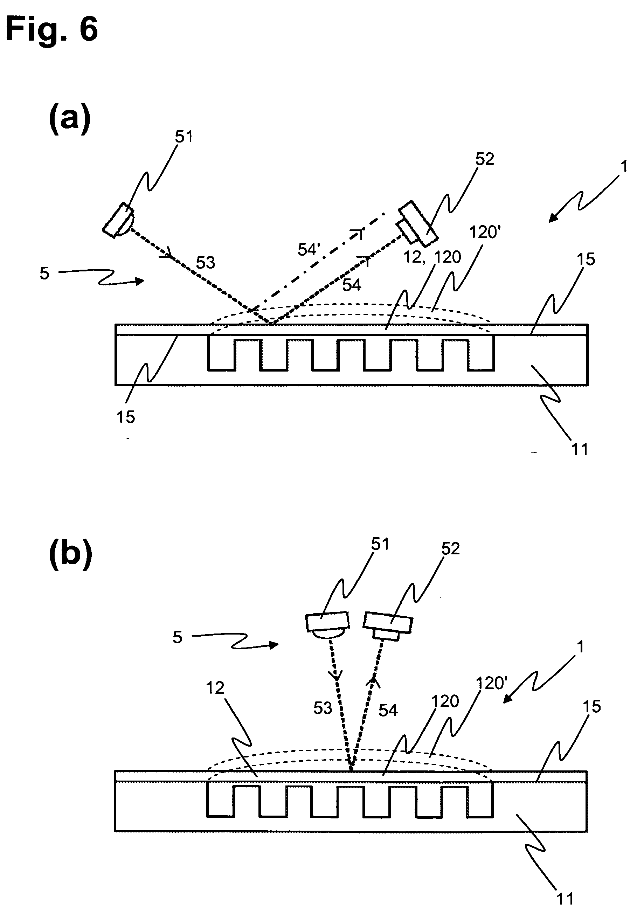

There are different possibilities to measure the displacement of the flexible membrane 120. Two possible embodiments of detection systems 5 based on optical principles are shown in **Figure 6**. In Figure 6(a) a cross-section of a micro-fluidic chamber 1 similar to Figure 1 is shown. The top cover 12 is realized as a flexible, resilient membrane layer 120, sealed 15 to the basic structure 11 along the outer rim of the chamber 1. An optical detection system 5 comprises a light emitting device 51, such as for example a light emitting diode (LED) or a laser diode, and a photo sensor 52, such as for example a photo diode or a photo transistor. The light emitting device 51 and the photo sensor 52 are arranged such that an incident light beam 53 emitted by the light emitting device 51 is reflected 54 by the surface of the top cover 12 toward the photo sensor 52, where it is detected. The top cover may be metal vapor coated to increase reflection. When the top cover 12 bulges under a positive pressure difference (dashed lines 120') the reflected light beam 54' does not impinge any longer on photo sensor 52. In such an embodiment the detection system 5 thus delivers a binary on/off signal correlated to a certain pressure threshold, which can be used by a control unit of an infusion pump system. Such a relatively simple system is completely sufficient to detect occlusion in a fluid line. To achieve a higher resolution in the pressure values, a sensor array can be used instead of a single sensor. This embodiment is preferable if the pressure values are used by a control unit to calculate the current flow of liquid and the administered dose of liquid medicament

Another variant of an optical detection system is shown in Figure 6(b), where the light emitting device 51 and the photo sensor 52 are arranged in such a way that the reflected light beam 54 will fall onto the photo sensor 52 independent of a displacement of the top cover membrane 120, 120'. The position of the surface of the top cover 12 is determined by analyzing the amplitude of the reflected light 54, which depends to the length of the combined light path 52, 54.

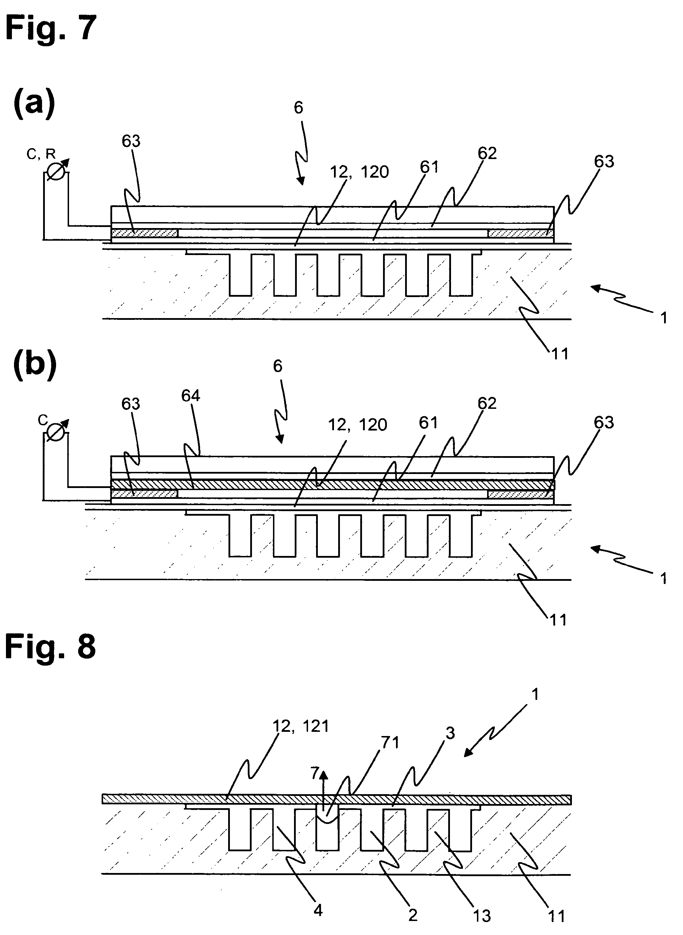

Further advantageous embodiments of a pressure sensor with a micro-fluidic chamber 1 according to the invention are shown in Figure 7, where the displacement of the flexible membrane 120 is determined by measuring a capacitance. In the embodiment of Figure 7(a), a first capacitor electrode 61, for example a thin metal foil, is arranged adjacent to the flexible top cover membrane 120. Alternatively the membrane 120 itself can be realized as a capacitor electrode 61, for example by coating it with a conducting material.

Capacitive pressure sensors are disclosed in more detail in European patent application 09003798.7 of the applicants. The disclosure of said application is incorporated herein by reference in its entirety. A micro-fluidic chamber according to the invention can be applied for such a sensor.

In the embodiment in Figure 7 insulating spacer elements 63 define a distance between said first capacitor electrode 61 and a second capacitor electrode 62, located on top of the spacer elements 63 and the first capacitor electrode 61. The two capacitor electrodes 61, 62 are electrically isolated from each other, and thus act as a capacitor with a capacitance C, which can be measured. With increasing internal pressure in the micro-fluidic chamber 1, the flexible, resilient membrane 120 bulges outwards. The first capacitor electrode 61 is displaced towards the second capacitor electrode 62. As a result the capacitance C increases, which can be detected and used to determine the deformation of the membrane 12, 120, and the internal pressure in the chamber 1 causing said deformation, respectively.

When the internal pressure is high enough, the first capacitor electrode 61 will eventually touch the second capacitor electrode 61, and the ohmic resistance R between the two layers drops to zero. This event can also be detected by suitable electronic means, and can be used - in addition or as an alternative to the capacitance - as an input for a control system of an infusion pump device.

Another variant of such a capacitive detection system 6 is shown in Figure 7(b), where an additional insulating layer 64 is arranged between the spacer elements 63 and the second capacitor electrode 62. Said additional layer 64 inhibits a short-circuit between the two capacitor electrodes 61, 62, which can be preferable, depending on the used capacitance measurement circuitry.

In yet another advantageous embodiment, the second capacitor electrode 62 can be located on the opposite side of the chamber 1, below the bottom structure, or integrated into the bottom structure.

### Degassing device

Another advantageous application of micro-fluidic chambers according to the invention are degassing devices for fluidic systems; particularly in infusion pump devices for liquid medicaments. A possible embodiment of a degassing device according to the present invention with a micro-fluidic chamber 1 is shown in **Figure 8**, comprising a rigid bottom structure 11 and a gas-permeable membrane 121 as the top layer 12. The gas permeable membrane 121 has preferably a high stiffness. Such a degassing device can be used for example in a fluidic system of an infusion pump device, where it can be arranged in different positions along the liquid stream.

Prior to first use the degassing device with a micro-fluidic chamber according to the invention can be filled without air bubbles, independent on the orientation of the device in space, due to the advantageous characteristics of the micro-fluidic chamber 1. When later during the operational use of the fluidic system an air bubble 71 is flushed into the degassing device, along with the stream of liquid 4, it will move along the meandering fluid channel 2. The interface tensions will inhibit the entrance even of small air bubbles into the gap 3. However, the air 7 in the air bubble 71 will be able to pass the gas-permeable membrane 121, provided that there is a sufficiently high difference of the partial pressure of the gas between the fluidic system and the other side of the gas permeable membrane. For an air bubble in the fluidic system the partial gas pressure is essentially identical to the liquid pressure.

When the surface characteristics of the walls 13, the bottom structure 11, and the gas permeable membrane 121 are properly chosen, it is energetically preferable for an air bubble 71 to be in contact with the gas permeable membrane 121. However, it is also possible that an air bubble is drained through the gas permeable membrane indirectly, via the liquid, since the gas can be solved in the liquid. This effect is especially important for small air bubbles with a large surface compared to their volume. Although the air bubbles 71 cannot enter the gap 3, gas 7 solved in the liquid will migrate into the gap 3, and permeate through the membrane 121. Thus a degassing device with a micro-fluidic chamber 1 according to the invention has a larger effective membrane surface compared to the operational internal volume than known from the state of the art, which correlates to a faster dissolution of air bubbles. Another advantage of such degassing devices is the considerably reduced dead volume.

To leave the air bubbles more time to drain through the gas-permeable membrane, while at the same time maintaining a sufficiently high through-put of liquid, it is advantageous to combine such a degassing device according to the invention with a bubble trap. European patent application No. 09155216.6 of the applicants shows a number of advantageous embodiments of bubble traps that can be combined with degassing devices with micro-fluidic chambers according to the invention.

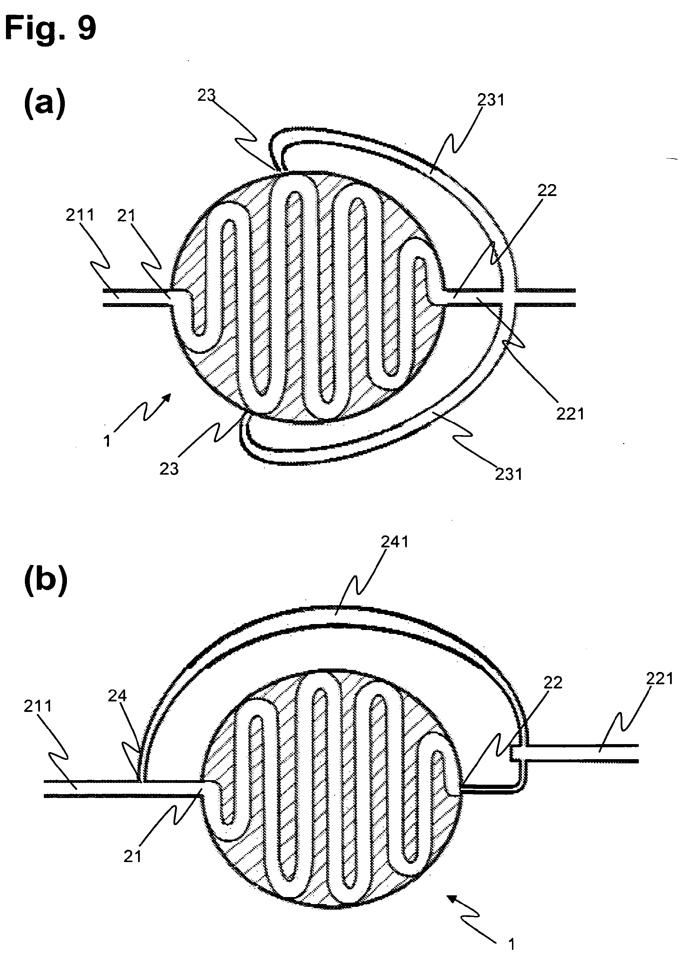

Two other particularly preferred embodiments of degassing devices according to the invention are disclosed in **Figure 9**. In Figure 9(a) two additional outlet openings 23 are located at different positions along the meandering fluid channel 2, from which additional outlet conduits 231 branch off. Downstream of the main outlet 22 the outlet conduits 221, 231 converge again to a common outlet conduit. The additional outlet openings 23 are smaller than the main outlet opening 22. The width of the narrow outlets 23 should be 50% or less of the width of the fluid channel 2. For an air bubble with certain dimensions is will be energetically less favorable to enter the narrow outlet 23, due to the interface tensions, than to stay in the comparably wide fluid channel 2. There the bubbles can continue to drain through the gas-permeable membrane. Thus the chamber 1 acts act as a bubble trap. Since three outlets 23, 22 are available, the through-put of liquid through the degassing device is increased.

Figure 9(b) depicts another preferred variant of a degassing device according to the invention with a micro-fluidic chamber 1, with an additional conduit 241 bypassing the chamber 1. Said bypass conduit 241 directly connects the inlet conduit 221 to the outlet conduit 221. The width of the inlet 24 of the bypass conduit 241 is much smaller, 50% or less, than the width of the inlet conduit 211. It is thus not favorable for an air bubble to enter the inlet 24 and bypass conduit 241, and the air bubble will enter the degassing device, where it can dissolve. In the shown embodiment the outlet 22 of the micro-fluidic chamber 1 is embodied narrower than in the previous embodiments discussed so far. It thus acts as a bubble trap, keeping any air bubble in the degassing device. Preferably the width of the outlet 22 in this embodiment should be 50% or less of the width of the fluid channel 2. To ensure that both the bypass conduit 241 and the micro-fluidic chamber 1 are completely filled during the first filling procedure, a flow barrier or a similar means can be used, as already discussed previously.

The additional outlets 231 in Figure 9(a) and the bypass 241 in Figure 9(b) are also useful in a pressure sensor according to the invention, in order to increase the overall capacity of the fluidic system.

The present invention is not to be limited in scope by the specific embodiments described herein. Indeed, various modifications of the present invention, in addition to those described herein, will be apparent to those skilled in the art from the foregoing description and accompanying drawings. Thus, such modifications are intended to fall within the scope of the appended claims.

### List of Reference Numerals

- 1: micro-fluid chamber
- 11: bottom substrate
- 12: top cover, top layer
- 120, 120': flexible, resilient membrane
- 121: gas-permeable membrane
- 13: wall, filling
- 14: inner volume
- 131: top surface of a wall
- 14: inner volume
- 15: sealing area
- 2, 2': fluid channel
- 21: inlet
- 211: inlet conduit
- 22: outlet
- 221: outlet conduit
- 23: additional outlet
- 231: additional outlet conduit
- 24: inlet of the bypass conduit
- 241: bypass conduit
- 3: fluid gap
- 3.1, 3.2, ...., 3.10: sections of the gap
- 4: liquid
- 5: optical detection system
- 51: light emitting device
- 52: photo sensor
- 53: incident beam
- 54, 54': reflected beam
- 6: capacitance detection system
- 61: first capacitor electrode
- 62: second capacitor electrode
- 63: spacer elements
- 64: insulation layer
- 7: air, gas
- 71: air bubble
- H1: height of the fluid chamber
- H2: height of a wall
- H3: height of the gap

## Claims

1. A micro-fluidic chamber (1) for use in a liquid medicament delivery system, having a bottom substrate (11) and a top cover (12), the top cover (12) being spaced from the bottom substrate (11) so as to define a height (H1) of the chamber (1), one or more walls or fillings (13) positioned in the chamber (1), the walls or fillings (13) defining one or more fluid channels (2) within the chamber, such that the fluid channel (2) extends from an inlet (21) of the chamber to an outlets (22) of the chamber, wherein the inlet and the outlet are openings positioned on the circumference of the chamber between the bottom substrate and the top cover, **characterized in that** the one or more fluid channels (2) have the height (H1) of the chamber, and each of the walls or fillings (13) has a height (H2) less than the height (H1) of the chamber (1) so as to define a fluid gap (3) between a top surface (131) of each wall or filling (13) and the top cover (12), and wherein the dimensions (H1, H2) of the walls or fillings (13) and the chamber (1) are chosen such that the fluid gap (3) will be filled with liquid (4) by capillary forces via the fluid channel (2) when liquid (4) is introduced into the fluid chamber (1).

2. The micro-fluidic chamber according to claim 1, **characterized in that** the top cover is a flexible, resilient membrane (120, 120'), and/or a gas-permeable membrane (121).

3. The micro-fluidic chamber according to any of the preceding claims, **characterized in that** at least a part of the surface of the bottom structure (11), and/or the walls (13), and/or the top cover (12) facing toward an inner volume (14) of the chamber (1) is hydrophilic.

4. The micro-fluidic chamber according to any of the preceding claims, **characterized in that** a height (H3) of the gap (3) lies between 0.02 and 0.2 mm, and more preferred between 0.05 and 0.15 mm.

5. The micro-fluidic chamber according to any of the preceding claims, **characterized in that** the fluid channel (2) has a meander-like shape.

6. The micro-fluidic chamber according to any of the preceding claims, **characterized by** two or more fluid channels (2, 2').

7. The micro-fluidic chamber according to any of the preceding claims, **characterized by** an additional conduit (241) bypassing the chamber (1) and fluidly connecting an inlet conduit (211) and an outlet conduit (221) of the chamber (1).

8. The micro-fluidic chamber according to claim 7, **characterized in that** the width of an inlet (24) of the bypass conduit (241) is smaller than the width of the inlet conduit (221).

9. The micro-fluidic chamber according to any of the preceding claims, **characterized in that** one or more additional outlets conduits (231) branch off from the fluid channel (2).

10. A pressure sensor for use in a liquid medicament delivery system, **characterized by** a micro-fluidic chamber (1) according to any of claims 1 to 9.

11. The pressure sensor according to claim 10, **characterized by** a detection system (5, 6) that is arranged to measure a deformation of a top cover (12, 120) of the micro-fluidic chamber (1).

12. A degassing device for use in a liquid medicament delivery system, **characterized by** a micro-fluidic chamber (1) according to any of claims 1 to 9.

13. An infusion pump device for use in a liquid medicament delivery system, **characterized by** a micro-fluidic chamber according to any of claims 1 to 9, and/or a pressure sensor according to claim 10 or 11, and/or a degassing device according to claim 12.

14. A liquid medicament delivery system, **characterized by** a micro-fluidic chamber according to any of claims 1 to 9, and/or a pressure sensor according to claim 10 or 11, and/or a degassing device according to claim 12.

## Patentansprüche

1. Mikrofluidische Kammer (1) zur Verwendung in Flüssigmedikament-Verabreichungssystemen, umfassend ein unteres Substrat (11) und eine obere Abdeckung (12), wobei die obere Abdeckung (12) von dem unteren Substrat (11) beabstandet ist, so dass eine Höhe (H1) der Kammer (1) definiert wird, eine oder mehrere in der Kammer (1) angeordnete Wände oder Einlagen (13), wobei die Wände oder Einlagen (13) einen oder mehrere Fluidkanäle (2) in der Kammer definieren, so dass sich der Fluidkanal (2) von einem Einlass (21) der Kammer zu einem Auslass (22) der Kammer erstreckt, wobei der Einlass und der Auslass Öffnungen sind, welche an dem Umfang der Kammer zwischen dem unteren Substrat und der oberen Abdeckung angeordnet sind, **dadurch gekennzeichnet, dass** der eine oder die mehreren Fluidkanäle (2) die Höhe (H1) der Kammer aufweisen, und jede der Wände oder Einlagen (13) eine Höhe (H2) aufweist, welche geringer ist als die Höhe (H1) der Kammer (1), so dass ein Fluidzwischenraum (3) zwischen einer oberen Oberfläche (131) jeder Wand oder Einlage (13) und der oberen Abdeckung (12) definiert wird, und wobei die Dimensionen (H1, H2) der Wände oder Einlagen (13) und der Kammer (1) derart gewählt werden, dass der Fluidzwischenraum (3) durch Kapillarkräfte über den Fluidkanal (2) mit Flüssigkeit (4) gefüllt wird, wenn Flüssigkeit (4) in die Fluidkammer (1) eingeleitet wird.

2. Mikrofluidische Kammer nach Anspruch 1, **dadurch gekennzeichnet, dass** die obere Abdeckung eine flexible, elastische Membran (120, 120') und/oder eine gasdurchlässige Membran (121) ist.

3. Mikrofluidische Kammer nach einem der vorhergehenden Ansprüche, **dadurch gekennzeichnet, dass** zumindest ein Teil der einem Innenvolumen (14) der Kammer (1) zugewandten Oberfläche der unteren Struktur (11) und/oder der Wände (13) und/oder der oberen Abdeckung (12) hydrophil ist.

4. Mikrofluidische Kammer nach einem der vorhergehenden Ansprüche, **dadurch gekennzeichnet, dass** eine Höhe (H3) des Zwischenraums (3) zwischen 0.02 und 0.2 mm und vorzugsweise zwischen 0.05 und 0.15 mm liegt.

5. Mikrofluidische Kammer nach einem der vorhergehenden Ansprüche, **dadurch gekennzeichnet, dass** der Fluidkanal (2) eine mäanderartige Form aufweist.

6. Mikrofluidische Kammer nach einem der vorhergehenden Ansprüche, **gekennzeichnet durch** zwei oder mehr Fluidkanäle (2, 2').

7. Mikrofluidische Kammer nach einem der vorhergehenden Ansprüche, **gekennzeichnet durch** eine zusätzliche Leitung (241), welche die Kammer (1) umgeht und eine Einlassleitung (211) und eine Auslassleitung (221) der Kammer (1) fluidisch verbindet.

8. Mikrofluidische Kammer nach Anspruch 7, **dadurch gekennzeichnet, dass** die Breite eines Einlasses (24) der Umgehungsleitung (241) geringer als die Breite der Einlassleitung (221) ist.

9. Mikrofluidische Kammer nach einem der vorhergehenden Ansprüche, **dadurch gekennzeichnet, dass** eine oder mehr zusätzliche Auslassleitungen (231) von dem Fluidkanal (2) abzweigen.

10. Drucksensor zur Verwendung in einem Flüssigmedikament-Verabreichungssystem, **gekennzeichnet durch** eine mikrofluidische Kammer (1) nach einem der Ansprüche 1 bis 9.

11. Drucksensor nach Anspruch 10, **gekennzeichnet durch** ein Detektionssystem (5, 6), welches zum Messen einer Verformung einer oberen Abdeckung (12, 120) der mikrofluidischen Kammer (1) eingerichtet ist.

12. Entgasungsvorrichtung zur Verwendung in einem Flüssigmedikament-Verabreichungssystem, **gekennzeichnet durch** eine mikrofluidische Kammer (1) nach einem der Ansprüche 1 bis 9.

13. Infusionspumpenvorrichtung zur Verwendung in einem Flüssigmedikament-Verabreichungssystem, **gekennzeichnet durch** eine mikrofluidische Kammer nach einem der Ansprüche 1 bis 9 und/oder einen Drucksensor nach Anspruch 10 oder 11 und/oder eine Entgasungsvorrichtung nach Anspruch 12.

14. Flüssigmedikament-Verabreichungssystem, **gekennzeichnet durch** eine mikrofluidische Kammer nach einem der Ansprüche 1 bis 9 und/oder einen Drucksensor nach Anspruch 10 oder 11 und/oder eine Entgasungsvorrichtung nach Anspruch 12.

## Revendications

1. Chambre micro-fluidique (1) destinée à être utilisée dans un système de délivrance de médicament liquide, comprenant un substrat inférieur (11) et un couvercle supérieur (12), le couvercle supérieur (12) étant espacé du substrat inférieur (11) de façon à définir une hauteur (H1) de la chambre (1), une ou plusieurs parois ou garnitures (13) positionnées dans la chambre (1), les parois ou garnitures (13) définissant un ou plusieurs canaux de fluide (2) à l'intérieur de la chambre, de telle sorte que le canal de fluide (2) s'étend depuis une entrée (21) de la chambre jusqu'à une sortie (22) de la chambre ; l'entrée et la sortie étant des orifices positionnés sur la circonférence de la chambre entre le substrat inférieur et le couvercle supérieur, **caractérisée en ce que** les un ou plusieurs canaux de fluide (2) ont la hauteur (H1) de la chambre, et chacune des parois ou garnitures (13) a une hauteur (H2) inférieure à la hauteur (H1) de la chambre (1) de manière à définir un espace de fluide (3) entre une surface supérieure (131) de chaque paroi ou garniture (13) et le couvercle supérieur (12), et les dimensions (H1, H2) des parois ou garnitures (13) et de la chambre (1) étant choisies de telle sorte que l'espace de fluide (3) sera rempli de liquide (4) par des forces capillaires par le biais du canal de fluide (2) lorsque le liquide (4) est introduit dans la chambre à fluide (1).

2. Chambre micro-fluidique selon la revendication 1, **caractérisée en ce que** le couvercle supérieur est une membrane élastique souple (120, 120'), et/ou une membrane (121) perméable aux gaz.

3. Chambre micro-fluidique selon l'une quelconque des revendications précédentes, **caractérisée en ce qu'**au moins une partie de la surface de la structure inférieure (11), et/ou les parois (13), et/ou le couvercle supérieur (12) tourné vers un volume intérieur (14) de la chambre (1) est hydrophile.

4. Chambre micro-fluidique selon l'une quelconque des revendications précédentes, **caractérisée en ce qu'**une hauteur (H3) de l'espace (3) est comprise entre 0.02 et 0.2 mm, et de manière plus préférée entre 0.05 et 0.15 mm.

5. Chambre micro-fluidique selon l'une quelconque des revendications précédentes, **caractérisée en ce que** le canal de fluide (2) a une forme de méandre.

6. Chambre micro-fluidique selon l'une quelconque des revendications précédentes, **caractérisée par** deux canaux de fluide (2, 2') ou plus.

7. Chambre micro-fluidique selon l'une quelconque des revendications précédentes, **caractérisée par** un conduit supplémentaire (241) contournant la chambre (1) et reliant de manière fluidique un conduit d'entrée (211) et un conduit de sortie (221) de la chambre (1).

8. Chambre micro-fluidique selon la revendication 7, **caractérisée en ce que** la largeur d'une entrée (24) du conduit de dérivation (241) est inférieure à la largeur du conduit d'entrée (221).

9. Chambre micro-fluidique selon l'une quelconque des revendications précédentes, **caractérisée en ce qu'**un ou plusieurs conduits de sortie supplémentaires (231) bifurquent du canal de fluide (2).

10. Capteur de pression destiné à être utilisé dans un système de délivrance de médicament liquide, **caractérisé par** une chambre micro-fluidique (1) selon l'une quelconque des revendications 1 à 9.

11. Capteur de pression selon la revendication 10, **caractérisé par** un système de détection (5, 6) qui est adapté pour mesurer une déformation d'un couvercle supérieur (12, 120) de la chambre micro-fluidique (1).

12. Dispositif de dégazage destiné à être utilisé dans un système de délivrance de médicament liquide, **caractérisé par** une chambre micro-fluidique (1) selon l'une quelconque des revendications 1 à 9.

13. Dispositif de pompe de perfusion destiné à être utilisé dans un système de délivrance de médicament liquide, **caractérisé par** une chambre micro-fluidique selon l'une quelconque des revendications 1 à 9, et/ou un capteur de pression selon la revendication 10 ou 11, et/ou un dispositif de dégazage selon la revendication 12.

14. Système de délivrance de médicament liquide, **caractérisé par** une chambre micro-fluidique selon l'une quelconque des revendications 1 à 9, et/ou un capteur de pression selon la revendication 10 ou 11, et/ou un dispositif de dégazage selon la revendication 12.
